# FASCICULE DE BREVET EUROPEEN

(11) **EP 4 545 947 B1**
(45) Date de publication et mention de la délivrance du brevet: **27.08.2025**
(21) Numéro de dépôt: 24202988.2
(22) Date de dépôt: 26.09.2024
(51) Int. Cl.: G01N 21/17, A01K 45/00, G01N 21/31, G01N 33/08, A01K 43/00

(54) **PROCÉDÉ ET SYSTÈME DE CARACTÉRISATION DU GENRE D'UN OEUF**
VERFAHREN UND VORRICHTUNG ZUR CHARAKTERISIERUNG DES GESCHLECHTS EINES EIES
METHOD AND SYSTEME FOR CHARACTERIZING THE GENDER OF AN EGG

(30) Priorité: 24.10.2023 FR 2311553
(43) Date de publication de la demande: 30.04.2025
(73) Titulaire: Commissariat à l'Energie Atomique et aux Energies Alternatives, 75015 Paris (FR); Ovalie Innovation, 32000 Auch (FR)
(72) Inventeur: REDON, Olivier, 38054 Grenoble cedex 09 (FR); MAKKI, Ihab, 38054 Grenoble cedex 09 (FR); CAILLY, Alexis, 38054 Grenoble cedex 09 (FR); MARTINEZ, Juan, 32000 AUCH (FR); LONJOU, Ilona, 32000 AUCH (FR); VERONESE, Thierry, 32000 AUCH (FR)
(74) Mandataire: INNOV-GROUP

(56) Documents cités:
- WO-A1-2021/145771
- WO-A1-2022/157760
- US-A1- 2012 318 981
- US-A1- 2020 400 640
- CORION MATTHIAS ET AL: "In ovo sexing of eggs from brown breeds with a gender-specific color using visible-near-infrared spectroscopy: effect of incubation day and measurement configuration", POULTRY SCIENCE, vol. 101, no. 5, 1 May 2022 (2022-05-01), Oxford, pages 101782, XP055962758, ISSN: 0032-5791, DOI: 10.1016/j.psj.2022.101782

## Description

### Domaine technique de l'invention

La présente invention concerne le domaine de la production aviaire et notamment la détermination non invasive du genre d'embryons d'œufs aviaires, en cours d'incubation.

### Etat de la technique

La recherche d'une technique de sexage précoce et non invasive des œufs de volailles est un sujet qui intéresse l'industrie volaillère depuis plusieurs années.

Des techniques invasives ont été proposées dans l'état de la technique, notamment dans les demandes de brevets WO98/14781, DE102007013102, WO2010/103111, US2011/0144473A1, WO2017/174337.

Des techniques optiques non invasives ont également été proposées mais elles concernent principalement la détection de la fertilité des œufs et non le sexage. Sur ce sujet, on peut notamment citer le brevet US 9,435,732 et le brevet US 6,029,080.

Dans la filière palmipède pour la production de foie gras, le canard mulard a été spécifiquement sélectionné car il résulte d'un croisement entre une cane de Pékin et un canard de Barbarie, porteur d'un gène albinos transmis uniquement aux femelles de type mulard. Cela permet de les sexer, après éclosion, par la couleur de l'œil : Les mâles ont en effet des yeux noirs car ils contiennent de la mélanine et les femelles albinos ont les yeux rouges. Cette particularité permet d'envisager un sexage in ovo autour du 9^{ème} jour en effectuant un mirage des œufs. En agitant l'œuf, il est alors possible d'amener l'œil au contact de la coquille ce qui permet de le distinguer.

Dans l'état de l'art, le mirage pour le sexage utilise une diode électroluminescente visible qui éclaire l'œuf par le haut, l'œuf étant positionné à 45°, et une caméra positionnée à 90° de l'axe de symétrie de l'œuf est destinée à capter l'image de diffusion de la lumière. Cette configuration n'est pas idéale car l'homogénéité de l'éclairage dans l'œuf est mauvaise. En effet, comme la lumière est diffusée dans toutes les directions, plus le chemin de la lumière dans l'œuf est long, plus l'absorption de la lumière est importante. On obtient dès lors un gradient d'intensité du haut vers le bas, qui est d'autant plus renforcé que la lumière doit traverser le jaune, celui-ci étant plus absorbant que le blanc. L'œil de l'embryon qui surnage à la surface du jaune apparait donc à la limite de la zone sombre, ce qui gêne sa détection. La forte absorption du jaune à ce stade de l'incubation vient du développement du système cardio-vasculaire qui contient de l'hémoglobine, présentant une très forte capacité d'absorption dans la gamme de longueurs d'ondes de l'éclairage de la diode électroluminescente entre 400 et 620nm.

Grâce à une combinaison de mirage et d'agitation autour du 9^{ème} jour, on parvient à observer l'œil chez les mâles, mais cette technique souffre de plusieurs inconvénients :
- Le mirage avec une diode électroluminescente et une caméra monochrome ne permet pas d'obtenir un bon contraste de l'œil par rapport au système cardio-vasculaire, ce qui est source d'erreurs de prédiction.
- Le processus d'agitation est souvent assez violent, ce qui peut être dommageable pour l'embryon et peut venir affecter le taux d'éclosabilité.
- Plus l'incubation avance, plus l'embryon a tendance à s'enfoncer dans le jaune, ce qui complique le processus d'agitation. Le mouvement généralement utilisé pour ramener l'œil à la surface est un mouvement angulaire autour de l'axe de l'œuf, ce qui complexifie son déploiement industriel.

La publication référencée *"*CORION MATTHIAS et AL : In ovo sexing of eggs from brown breeds with a gender-specific color using visible-near-infrared spectroscopy : effect of incubation day and measurement configuration", POULTRY SCIENCE, coL.101, n°5 ; 1 mai 2022 page 101782, XP055962758, Oxford*,* décrit également une technique de sexage des œufs de poule par analyse spectroscopique.

Ce document antérieur décrit une technique de sexage de la poule par l'eumélanine contenue en grande quantité dans les plumes des femelles. La solution est basée sur une analyse de données spectrales. Dans cette approche, on s'applique à rechercher les signatures spectrales les plus marquantes pour la prédiction du sexe.

Il existe peu de solutions antérieures capables de déterminer le sexe d'un embryon dans l'œuf de manière fiable car divers composés biologiques sont présents dans l'œuf (hémoglobine, mélanine, protéines, lipides,...) et absorbent du flux lumineux à différentes longueurs d'ondes avec des intensités différentes, rendant le diagnostic compliqué. Sans conditionnement particulier de l'œuf, il est difficile de détecter l'œil de l'embryon car il n'est souvent pas visible à travers la coquille.

De plus, dans la gamme des longueurs d'ondes du visible, l'absorption du flux lumineux par les différents composés biologiques rend la distinction de l'œil très complexe et difficile à automatiser.

Il existe donc un besoin de disposer d'un procédé fiable dont toutes les étapes concourent à la détermination du genre de l'embryon dans l'œuf. Le procédé doit être d'une mise en œuvre simple et suffisamment rapide.

Le procédé permet notamment de pouvoir caractériser un embryon à un stade le plus précoce possible. S'agissant des œufs de canard, une telle caractérisation est mise en œuvre avant le 10ème jour depuis le début de l'incubation. Plus généralement, la caractérisation est effectuée au premier tiers de la procédure d'incubation.

### Exposé de l'invention

Ce but est atteint par un procédé de caractérisation du genre de l'embryon d"un œuf à partir de la présence d'un organe au sein de l'embryon de l'œuf, comprenant des étapes de :
- Préparation de l'œuf, consistant à mettre l'œuf en position couchée sur un support, l'axe longitudinal de l'œuf étant parallèle audit support sur lequel il repose,
- Mise en attente de l'œuf ainsi positionné pour que l'embryon de l'œuf migre vers le haut de l'œuf,
- Agitation mécanique de l'œuf par rotation de l'œuf autour de son axe longitudinal, en vue d'optimiser le positionnement dudit organe au plus près de la coquille,
- Pendant l'étape d'agitation mécanique, émission à travers l'œuf, par une source lumineuse, d'un flux lumineux, et acquisition de plusieurs images de l'œuf,
- Analyse des images de l'œuf générées, la présence dudit organe étant révélée par contraste d'absorption entre les composantes constitutives de l'œuf contenant du sang et ledit organe de l'embryon contenant de la mélanine.

Selon une particularité, le procédé comporte une étape de filtrage du flux lumineux dans une bande spectrale étroite.

Selon une particularité, la bande spectrale étroite est comprise entre 650nm et 750nm. Avantageusement, la bande spectrale étroite est comprise entre 675nm et 725nm. Selon une réalisation particulière, l'étape de filtrage est mise en œuvre sur le flux lumineux émis par la source lumineuse, avant transmission dans l'œuf.

Selon une autre réalisation particulière, l'étape de filtrage est mise en œuvre sur le flux lumineux émis par la source lumineuse, après transmission à travers l'œuf et avant l'étape d'acquisition d'images de l'œuf.

Selon une autre particularité, l'étape d'agitation mécanique consiste à mettre en œuvre une séquence de rotation de l'œuf autour de son axe longitudinal, ladite séquence consistant au moins à faire tourner l'œuf sur lui-même autour de son axe longitudinal dans un sens sur une première plage angulaire non nulle, puis dans le sens opposé sur une deuxième plage angulaire non nulle.

Selon une autre particularité, la séquence de rotation est mise en œuvre en jouant sur quatre paramètres :
- Le sens de rotation de l'œuf ;
- La vitesse de rotation de l'oeuf ;
- L'accélération conférée lors de la rotation ;
- La plage angulaire de rotation de l'œuf ;

L'invention concerne également un système de caractérisation du genre de l'embryon d'un œuf à partir de la présence d'un organe au sein de l'embryon de l'œuf, employé pour mettre en œuvre le procédé tel que défini ci-dessus, le système comportant :
- Un poste de préparation de l'œuf, configuré pour mettre l'œuf en position couchée sur un support, l'axe longitudinal de l'œuf étant parallèle audit support sur lequel il repose,
- Un système d'agitation mécanique de l'œuf par rotation de l'œuf autour de son axe longitudinal, en vue d'optimiser le positionnement dudit organe au plus près de la coquille,
- Un système d'imagerie comprenant une source lumineuse apte à émettre un flux lumineux à travers l'œuf, et un dispositif d'acquisition de plusieurs images de l'œuf, le système d'imagerie étant actionné pour fournir des images de l'œuf pendant son agitation mécanique,
- Des moyens d'analyse des images de l'œuf générées, la présence dudit organe étant révélée par contraste d'absorption entre les composantes constitutives de l'œuf contenant du sang et ledit organe de l'embryon contenant de la mélanine.

Selon une particularité, le système comporte des moyens de filtrage du flux lumineux émis par la source lumineuse configurés pour filtrer ledit flux lumineux dans une bande spectrale étroite.

Selon une autre particularité, la bande spectrale étroite est comprise entre 650nm et 750nm.

Avantageusement, la bande spectrale étroite est comprise entre 675nm et 725nm. Selon une réalisation particulière, les moyens de filtrage sont agencés pour filtrer le flux lumineux émis par la source lumineuse, avant transmission dans l'œuf.

Selon une autre réalisation particulière, les moyens de filtrage sont agencés pour filtrer le flux lumineux émis par la source lumineuse, après transmission à travers l'œuf et avant acquisition d'images de l'œuf par le dispositif d'acquisition.

Selon une autre particularité, le système d'agitation mécanique est contrôlé pour mettre en œuvre une séquence de rotation de l'œuf autour de son axe longitudinal, ladite séquence consistant au moins à faire tourner l'œuf sur lui-même autour de son axe longitudinal dans un sens sur une première plage angulaire non nulle, puis dans le sens opposé sur une deuxième plage angulaire non nulle.

Selon une autre particularité, la séquence de rotation est mise en œuvre en jouant sur quatre paramètres :
- Le sens de rotation de l'œuf ;
- La vitesse de rotation de l'œuf ;
- L'accélération conférée lors de la rotation ;
- La plage angulaire de rotation de l'œuf ;

### Brève description des figures

D'autres caractéristiques et avantages vont apparaître dans la description détaillée qui suit, en liaison avec les figures annexées listées ci-dessous :
- La figure 1 montre de manière schématique la structure d'un œuf, montré en position horizontale ;
- Les figures 2A à 2C illustrent les étapes d'incubation, de transfert et de préparation des œufs ;
- La figure 3 illustre de manière schématique le principe de fonctionnement du système d'imagerie de l'invention ;
- La figure 4A montre un exemple de spectre d'émission de la source lumineuse employée et illustre la plage d'intérêt P centrée autour de 700nm et la figure 4B montre le spectre de transmission d'un filtre passe-bande utilisé pour sélectionner la plage d'intérêt ;
- La figure 5 montre un exemple de réalisation d'une installation employée pour caractériser plusieurs œufs de manière simultanée ;
- La figure 6 montre en vue de dessus, un exemple de réalisation d'un système d'agitation mécanique d'un œuf ;
- La figure 7 montre plusieurs photos capturées par la caméra du système de l'invention, à différents stades d'incubation de l'œuf, et permet d'illustrer l'intérêt du procédé et du système de l'invention ;

### Description détaillée d'au moins un mode de réalisation

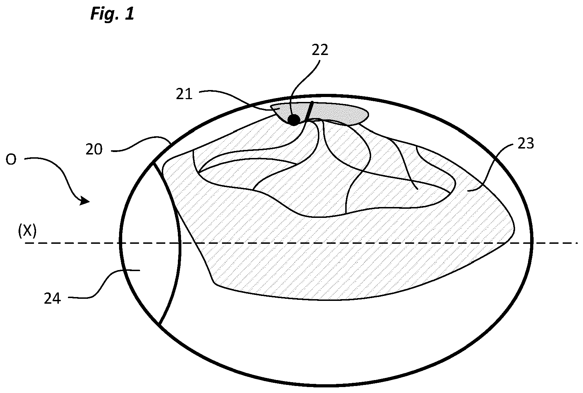

Dans la suite de la description et comme illustré par la figure 1, l'œuf O est défini par son axe de symétrie de révolution (axe (X)) dans la direction longitudinale et par sa surface de forme ovoïde. Son équateur correspond à la ligne imaginaire tracée sur sa surface sur toute sa circonférence, au niveau de sa section transversale la plus large.

Le système de l'invention est utilisé pour détecter la présence d'un organe d'un être vivant dans un œuf O et permet, in fine, de déterminer par exemple le sexe (mâle ou femelle) de l'être vivant ou l'état de fécondation. Il est notamment parfaitement adapté pour le sexage d'un œuf de canard de type mulard. Mais il peut également être utilisé pour déterminer la présence, voire le sexe, de tout autre embryon d'ovipare dont l'œil, la plume ou un autre organe présente une coloration par la mélanine.

Le procédé et le système de l'invention sont notamment basés sur une analyse par imagerie classique, et non par analyse de données spectrales.

De manière connue, en référence à la figure 1, l'œuf O comporte une coquille 20, dans laquelle est présent l'embryon 21 auquel appartient l'œil 22, et le système cardio-vasculaire 23 contenant notamment de l'hémoglobine. L'œuf comporte également une chambre ou poche à air 24 qui est généralement située du côté opposé de la pointe de l'œuf. Sur la figure 1, l'œuf O est représenté à l'horizontale, la chambre à air 24 positionnée sur le côté.

En vue de déterminer le genre d'un embryon dans l'œuf, le procédé décrit ci-dessous comporte les étapes principales suivantes :
- Incubation et transfert ;
- Préparation de l'œuf pour le placer dans une position déterminée et mise en attente de l'œuf dans cette position ;
- Agitation mécanique de l'œuf ;
- Pendant l'étape d'agitation mécanique, imagerie de l'œuf ;
- Analyse des images de l'œuf générées ;

### Incubation et transfert

### Figure 2A

### Figure 2B

Il faut noter que l'incubation est mise en œuvre de manière connue et classique.

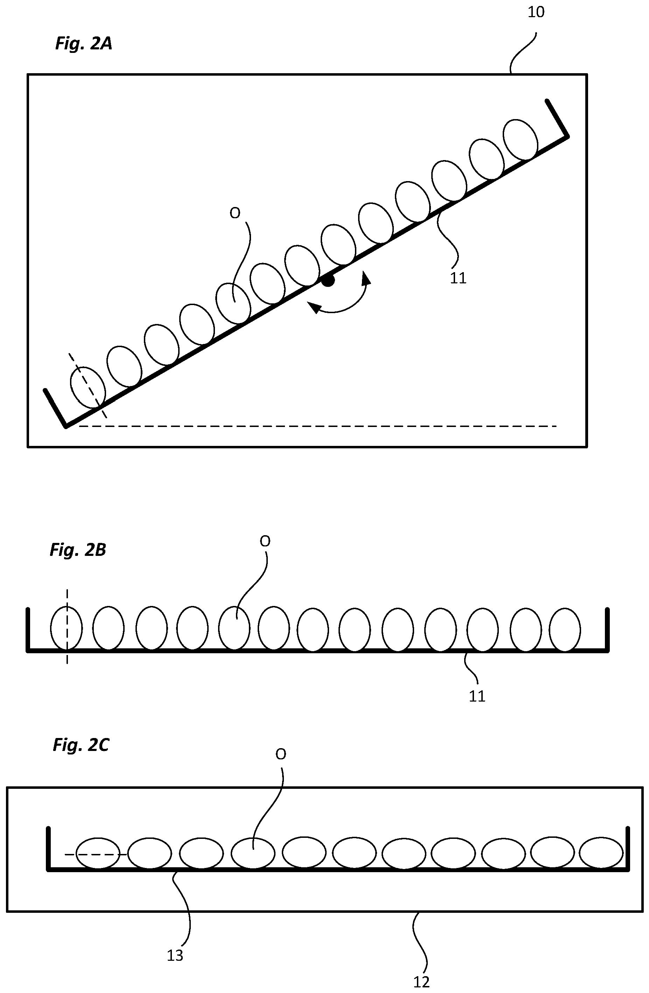

Lors de l'incubation (figure 2A), les œufs sont disposés sur des grilles d'incubation 11 dans un incubateur 10 ayant une atmosphère contrôlée notamment en température, et humidité. Classiquement, la température dans un incubateur est contrôlée autour de 37,5°C. Au cours de l'incubation, les œufs sont par exemple placés en position verticale, c'est-à-dire avec leur axe longitudinal orienté à 90° par rapport à la grille. Pendant l'incubation, comme illustré par la figure 2A, les grilles d'incubation peuvent basculer de 45° d'un côté ou de l'autre toutes les heures pour éviter à l'embryon 21 de coller à la coquille 20.

À la suite de l'incubation, pendant une certaine durée, les œufs sont transférés de l'incubateur vers un poste de préparation. En particulier, les grilles d'incubation 11 sont alors positionnées en position horizontale, parallèle au sol pendant le transfert. Pendant le transfert (figure 2B), les œufs sont maintenus immobiles en position verticale (90° - comme sur la figure 2B) ou légèrement incliné (45°).

### Préparation et mise en attente de l'œuf

### Figure 2C

Cette étape consiste à préparer l'œuf pour qu'il soit dans les meilleures conditions pour la caractérisation ultérieure.

Elle est mise en œuvre dans un poste de préparation 12, après une durée d'incubation dans un couvoir.

Le poste de préparation 12 est agencé pour maintenir l'œuf en position couchée, sur un support, l'axe longitudinal (X) de l'œuf O étant parallèle au support 13 sur lequel il repose. Un tel support 13 peut être une plaque, un tapis, un logement spécifique, ou équivalent. Ainsi, l'œuf O est changé de position par rapport à celle utilisée au cours du transfert. La position couchée permet aux constituants internes de l'œuf O de migrer vers une nouvelle position. En effet les constituants internes n'étant pas fixés à la coquille, tout changement de position de l'œuf entraîne un déplacement de ses constituants. Chaque œuf O est ainsi transféré de la position à 90° (ou 45°) à la position horizontale. Des moyens spécifiques peuvent être employés pour assurer ce transfert de position, sans dommage.

De manière avantageuse, les œufs O sont maintenus en position couchée dans une atmosphère contrôlée, notamment en température, afin de surtout éviter le refroidissement des œufs et ainsi améliorer la détectabilité des constituants.

Le jaune d'œuf ou vitellus va toujours flotter sur l'albumen étant donnée la différence de densité. Quant à l'embryon, il se déplacera vers le point le plus haut du jaune d'œuf. En conséquence, l'embryon migre toujours vers le point le plus haut de la coquille. Un tel maintien en position couchée dure entre 1minute et 30 minutes.

L'étape de préparation et de mise en attente permet d'améliorer in fine la caractérisation du genre de l'embryon dans l'œuf. La visibilité des constituants internes de l'œuf (qui sont par ailleurs connus) est en effet maximale en position couchée de l'œuf :
- La chambre à air 24 est une barrière physique. Elle se trouve généralement sur la base large de l'œuf (à l'opposé de la pointe de l'œuf sur son axe longitudinal). En position couchée, la chambre à air 24 reste fixe dans sa position et les éléments internes migrent dans une zone où la chambre à air 24 ne peut pas les masquer.
- La surface occupée par les constituants de l'œuf est supérieure en position couchée et les constituants internes de l'œuf O s'étalent davantage, augmentant ainsi leur visibilité lors de la caractérisation.

Ensuite, les œufs O sont amenés vers le système d'imagerie, toujours en position couchée, et immobile en position couchée. Idéalement, les œufs ne doivent pas tourner pendant le transfert du poste de préparation au système d'imagerie utilisé pour caractériser le genre de l'embryon dans l'œuf. Au cours de la caractérisation, l'œuf est positionné sur un support 4 en position couchée dans le même état que celui à l'issu de sa préparation.

### Système d'imagerie : Principe

### Figure 3

### Figure 4A

### Figure 4B

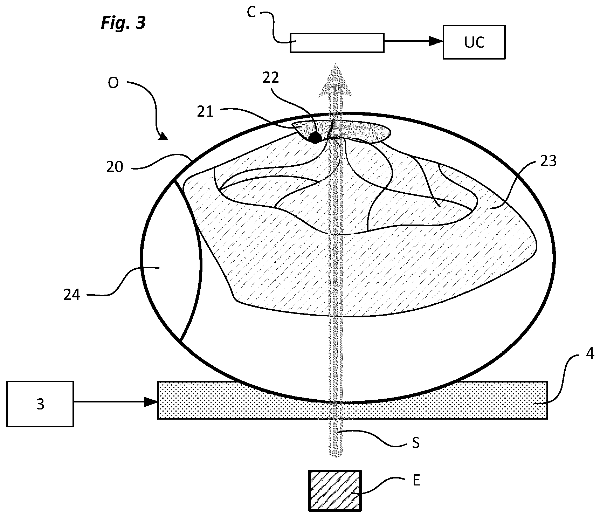

La figure 3 illustre le principe d'un système d'imagerie unitaire pour un seul œuf O, mais on verra que ce système peut être dupliqué pour un ensemble de plusieurs œufs.

Le système d'imagerie, appelé également système de mirage, comporte principalement une source lumineuse E et un capteur d'images C (caméra par exemple).

La source lumineuse E est par exemple constituée d'une ou plusieurs diodes électroluminescentes, positionnées pour éclairer l'œuf O. La source lumineuse E est avantageusement positionnée pour éclairer l'œuf O par le dessous et le dispositif d'acquisition d'images C est avantageusement placé au-dessus, suivant le même axe, afin de faire une acquisition par transmission. Dans ce cas, il faut noter que le support 4 de l'œuf O doit être choisi pour laisser passer le signal lumineux S émis par la source lumineuse. Il peut ainsi être choisi transparent pour laisser passer par exemple au moins 90% du signal lumineux, avec un minimum de diffusion, et/ou par exemple avec une ouverture par laquelle le signal lumineux S est émis, et/ou présenter au moins une partie en forme de grille destinée à être traversée par le signal.

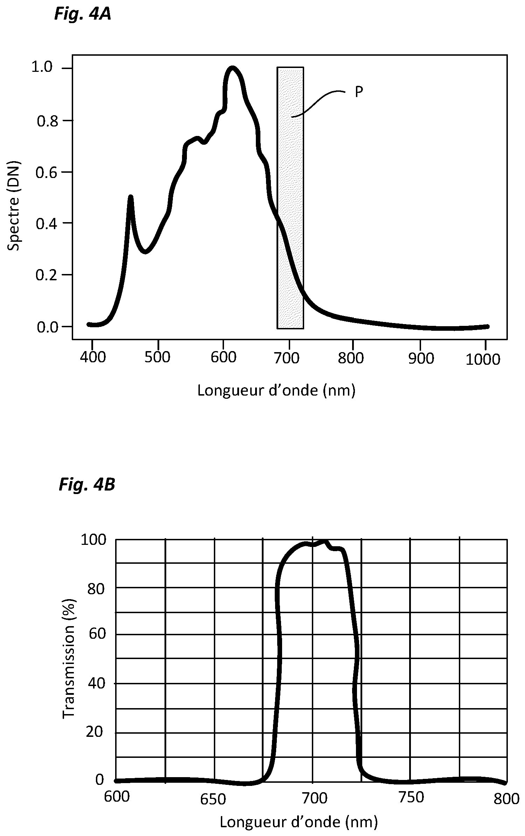

De manière non limitative, la source lumineuse E est par exemple choisie pour émettre avec un spectre tel que celui représenté par la courbe de la figure 4A.

Sur cette figure 4A, la plage d'intérêt P autour de 700nm est identifiée.

La figure 4B montre un exemple de réponse de filtre de type passe-bande autour de la plage d'intérêt de 700nm, qui peut être appliqué sur la source lumineuse E (parcours de la lumière en amont de l'œuf O) ou devant le capteur d'images C (parcours de la lumière en aval de l'œuf O). Préférentiellement, on choisira un filtre passe-bande ou en ensemble de filtres passe-haut et passe-bas qui définissent une fenêtre de transmission de 50nm de largeur entre 675nm et 725nm afin d'optimiser le contraste entre la mélanine et l'oxyhémoglobine contenues dans l'embryon. Plus la fenêtre spectrale s'élargira, plus le contraste diminuera, mais plus le flux reçu par le capteur d'images C sera important. Avantageusement, on ne dépassera pas une largeur de fenêtre de 100nm entre 650nm et 750nm pour maintenir un contraste d'absorption d'au moins 10 entre la mélanine et l'oxyhémoglobine.

Avantageusement, la ou les diodes électroluminescentes de la source lumineuse E auront une température de couleur qui permet une émission suffisante autour de 700nm. Dans le même temps, on privilégiera des diodes électroluminescentes qui ont une forte puissance lumineuse pour permettre un bon contraste dans des œufs ayant entre 7 et 10 jours d'incubation.

Le système d'imagerie, tel que présenté sur la figure 3, comporte également un capteur d'images C, par exemple une caméra, utilisée pour acquérir des images de l'œuf éclairé. La caméra est par exemple de type monochrome, de manière à pouvoir bien identifier le contraste entre les zones de l'œuf O où la lumière est transmise et les zones de l'œuf O où la lumière est absorbée.

Préférentiellement, le capteur d'images C est pourvu d'un filtre spectral à bande étroite centré sur la longueur d'onde 700nm. Comme indiqué ci-dessus, ce filtre permet d'éliminer les longueurs d'onde pour lesquelles des constituants de l'œuf O, notamment le système cardio-vasculaire contenant de l'oxyhémoglobine, ont une forte absorption qui nuit à la détection de la mélanine contenue dans les yeux des canards mulard mâles. La longueur d'onde à 700nm est celle où l'on a le maximum de différence d'absorption entre la mélanine et l'oxyhémoglobine. On privilégiera les filtres centrés à 700nm et ayant une faible largeur de bande (20 à 40nm), comme celui de la figure 4B. Des largeurs de bande plus grandes sont possibles mais au détriment de la qualité de détection de l'œil.

Le filtre est préférentiellement positionné entre l'œuf O et le capteur d'images C, ce qui permet d'observer uniquement dans la bande d'intérêt et donc d'éliminer également tout rayonnement parasite venant de l'environnement. Il pourrait cependant être positionné ailleurs, par exemple entre la source lumineuse et l'œuf. Avantageusement, le capteur d'images C est placé dans l'axe de la source lumineuse E.

A titre d'exemple, une combinaison de deux filtres peut être employée, un filtre passe-bas qui laisse passer les longueurs d'ondes inférieures à 720nm et un filtre passe-haut qui laisse passer les longueurs d'ondes supérieures à 670nm.

La solution de l'invention s'appuie donc notamment sur la capacité d'absorption de la lumière visible par la mélanine présente à l'intérieur de l'œuf O pour créer un contraste suffisant sur l'image capturée par le capteur d'images C.

En ce qui concerne le sexage d'un œuf O de canard mulard, l'utilisation d'une plage étroite de longueurs d'ondes autour de 700nm permet d'avoir un contraste d'absorption maximal entre la mélanine contenue dans les yeux des embryons mâles et les autres constituants biologiques de l'œuf, notamment avec l'hémoglobine du système cardio-vasculaire, cette dernière présentant un minimum d'absorption autour de 700nm. Par ce procédé, le système cardio-vasculaire 23 disparait quasiment de l'image obtenue au niveau du capteur, permettant une identification quasiment systématique de l'œil des embryons mâles et donc l'identification de son sexe dans l'œuf.

Il faut noter qu'il est également possible d'utiliser des diodes électroluminescentes émettant sur une bande spectrale étroite, centrée autour de 700nm, ce qui permet de s'affranchir du système de filtrage tout en conservant la fonctionnalité d'élimination des constituants non désirables pour la détection de l'œil. Pareillement, il est possible d'utiliser des lampes halogènes comme source lumineuse E en combinaison avec une filtre passe-bande autour de 700 nm.

Le filtrage à 700nm peut également être décalé à une autre longueur d'onde où les coefficients d'absorption de la mélanine et de l'oxyhémoglobine diffèrent suffisamment, comme cela est par exemple le cas autour de 435nm. Il est également possible d'utiliser une combinaison (linéaire, opération mathématique, etc.) de plusieurs longueurs d'ondes dont les coefficients d'absorption de la mélanine et de l'oxyhémoglobine diffèrent. Par exemple, le rapport entre une image prise à 435nm et une autre prise à 700nm peut être utilisé afin d'accroitre encore le contraste dans l'image entre l'œil contenant de la mélanine et le système cardio-vasculaire contenant de l'oxyhémoglobine.

### Système complet de caractérisation du genre des œufs

### Figure 5

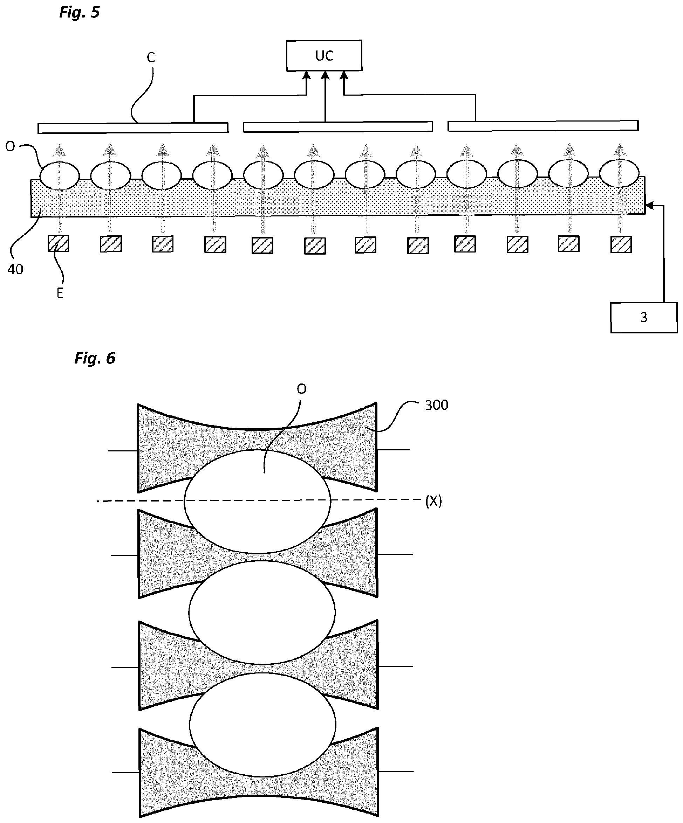

En référence à la figure 5, une installation complète permet de traiter plusieurs œufs O en même temps. Un plateau support 40 peut disposer de plusieurs emplacements distincts, destinés chacun au support d'un œuf O distinct à caractériser. Une source lumineuse E distincte peut être employée par emplacement et donc par œuf O à caractériser. Un même capteur d'images C peut être commun à plusieurs œufs à caractériser comme par exemple pour seize œufs (4x4) sur l'installation représentée sur la figure 5. Les moyens de traitement UC sont ensuite chargés de traiter les images qui ont été capturées par chaque capteur d'images C de l'installation, selon les principes définis ci-dessus.

Un système 3 d'agitation mécanique (voir ci-après) est associé au plateau, afin de repositionner l'embryon 21 de chaque œuf O vers le haut et assurer ainsi qu'il soit au plus près de la coquille et donc du capteur d'images C lors de la lecture du flux lumineux transmis.

### Système d'agitation mécanique

### Figure 3

### Figure 5

### Figure 6

L'agitation mécanique de l'œuf permet de positionner l'œil 22 de l'embryon au plus près de la coquille 20 pour le rendre le plus visible possible par le système d'imagerie. L'agitation mécanique est réalisée sur le même poste que celui utilisé pour l'imagerie (voir ci-après), le système d'imagerie étant alors actif pour acquérir des images alors que l'agitation mécanique de l'œuf O est en cours.

Sur la figure 3, le système 3 d'agitation mécanique est schématisé pour un poste unitaire et sur la figure 5, ce système 3 est schématisé pour un ensemble de plusieurs œufs O à caractériser.

Pour agiter mécaniquement les œufs, le système 3 comporte par exemple des rouleaux 300 en forme de diabolos actionnés par des moteurs, entre lesquels les œufs O sont positionnés. En faisant tourner ces rouleaux 300, on fait rouler les œufs sur eux-mêmes autour de leur axe longitudinal X. Des patins peuvent être prévus sur les rouleaux 300 pour adhérer à la surface de chaque œuf O.

Un exemple d'une telle architecture à rouleaux est représenté sur la figure 6.

Le procédé d'agitation spécifique permet d'optimiser le positionnement de l'œil 22 de l'embryon au plus près de la coquille 20, permettant une amélioration de sa détection par le système d'imagerie.

L'agitation mécanique peut prendre plusieurs formes. Il s'agit de jouer sur quatre paramètres :
- Le sens de rotation de l'œuf ;
- La vitesse de rotation de l'œuf ;
- L'accélération conférée lors de la rotation ;
- L'angle de rotation de l'œuf ;

Il faut noter que les différences de vitesse et d'accélération produisent des résultats différents pour la visibilité de l'embryon :
- Une vitesse moyenne et une accélération faible permettent de conserver l'embryon proche de la coquille 20 tout le long du mouvement ;
- Une vitesse et une accélération fortes projettent l'embryon vers la coquille et permettent de l'apercevoir durant quelques images ;
- Les mouvements plus intenses (allers-retours secs) sont brefs afin de ne pas endommager l'embryon ;

Une pré-agitation peut être menée afin de rapprocher l'embryon et son œil près de la surface interne de la coquille 20. Cette pré-agitation peut consister à faire tourner l'œuf O sur lui-même autour de son axe sur 360°, à vitesse faible (environ 0.8 tour par seconde) durant quelques secondes (entre 5 secondes et 12 secondes).

Une pause d'une seconde peut ensuite être intégrée dans le processus avant de procéder à l'agitation mécanique proprement dite.

Les séquences d'agitation mécanique de l'œuf O décrites ci-dessous ont mené chacune à des résultats probants, en vue de la caractérisation du genre de l'œuf O via le système d'imagerie décrit ci-dessus. Cependant, celles-ci sont à considérer de manière non limitative. Il est bien entendu possible de faire varier les angles de rotation et les vitesses de rotation, sans altérer les résultats de manière significative. La caméra du système d'imagerie est placée au-dessus de l'œuf O, l'œuf étant positionné à plat suivant son axe longitudinal (X) par rapport à l'axe de la caméra. Dans la description de la séquence, un aller-retour désigne une rotation de l'œuf O sur lui-même autour de son axe longitudinal (X), dans un sens de rotation (sens anti-horaire choisi par exemple comme le sens positif) sur un premier angle de rotation, puis dans le sens opposé (sens horaire choisi comme le sens négatif) sur un deuxième angle de rotation (pas forcément identique au premier angle de rotation).
Première séquence :
   - Un premier aller-retour sur un angle de +67.5° puis sur -135°, puis
   - Trois allers-retours consécutifs en effectuant des mouvements de -135° et +135°, couvrant une surface qui s'étend entre +67,5° et -67,5° par rapport à la position initiale (angle total de 135°),
   - de +135 ° à -135°, à vitesse moyenne et accélération faible, puis
   - Retour en position initiale (à 0°) puis demi-tour de 180 ° de l'œuf, puis
   - A nouveau :
   - Un premier aller-retour sur un angle de +67,5° puis sur -135°, puis
   - trois allers-retours consécutifs sur la plage allant de +135 ° à -135°, à vitesse moyenne et accélération faible
Deuxième séquence :
   - Un aller-retour entre la position initiale et +135°, à vitesse et accélération fortes, puis
   - Un aller-retour dans le sens opposé entre la position initiale et -135 °, à vitesse et accélération fortes, puis
   - Un premier aller-retour sur un angle de +90° puis sur un angle de -180°, puis
   - Un aller-retour sur un angle total de 180 ° à vitesse moyenne et accélération faible, puis
   - Retour en position initiale, puis demi-tour de l'œuf sur 180°, puis
   - Un aller-retour entre la position initiale et +135°, à vitesse et accélération fortes, puis
   - Un aller-retour dans le sens opposé entre la position initiale et -135 °, à vitesse et accélération fortes, puis
   - Un premier aller-retour sur un angle de +90° puis sur -180°, puis
   - Un aller-retour sur un angle total de 180 ° à vitesse moyenne et accélération faible.
Troisième séquence :
   - Un premier aller-retour sur un angle de +90° puis sur -180°, puis
   - Un aller-retour sur un angle total de 180° à vitesse moyenne et accélération faible, puis
   - Un aller-retour entre la position initiale et +135°, à vitesse et accélération fortes, puis
   - Un aller-retour dans le sens opposé entre la position initiale et -135° à vitesse et accélération fortes, puis
   - Retour en position initiale, puis demi-tour de l'œuf de 180°, puis
   - Un premier aller-retour sur un angle de+90° puis sur -180°, puis
   - Un aller-retour sur un angle total de 180° à vitesse moyenne et accélération faible, puis
   - Un aller-retour entre la position initiale et +135°, à vitesse et accélération fortes, puis
   - Un aller-retour dans le sens opposé entre la position initiale et -135 °, à vitesse et accélération fortes.

Pendant le procédé d'agitation des œufs, la caméra du système d'imagerie enregistre des vidéos des œufs selon le principe de mirage décrit précédemment. Ces banques d'images vont servir à la classification du genre de l'embryon.

### Traitement des images

Le traitement des images acquises par le capteur d'images C est réalisé par des moyens de traitement UC.

De manière non limitative, le traitement peut être effectué en entraînant un premier réseau de neurones sur une base de séquences vidéo, elles-mêmes décomposées en sous-séquences vidéos de quelques images (en pratique de 1 à 6 images), ces sous-séquences vidéo étant labellisées de façon semi-supervisée par un algorithme de traitement d'image qui sélectionne les images ayant la plus forte probabilité de présenter un œil visible (la mise en place d'un tel algorithme est facilité par l'efficacité du système à révéler la présence de l'œil dans l'image). Ce réseau de neurones peut également être utilisé comme outil de labellisation semi-supervisée pour l'apprentissage d'un nouveau modèle. En ce but, une nouvelle base de données d'apprentissage est ainsi construite en prenant l'ensemble des séquences de 1 à 6 images pour les femelles et uniquement les séquences de 1 à 6 images sélectionnées par le premier réseau de neurones pour les mâles. On s'assure ainsi de ne prendre dans cette nouvelle base d'apprentissage que des séquences vidéo ayant une très forte probabilité de présence de l'œil pour les mâles. Avec cette seconde itération, les performances de prédiction atteignent 99% pour les mâles et 97% pour les femelles. Cette approche semi-supervisée pour la création de nouvelles bases de données et l'apprentissage de nouveaux modèles peut être réitérée autant de fois que nécessaire pour atteindre les performances de prédiction souhaitées pour le modèle.

D'autres solutions de traitement pourraient bien entendu être mises en œuvre.

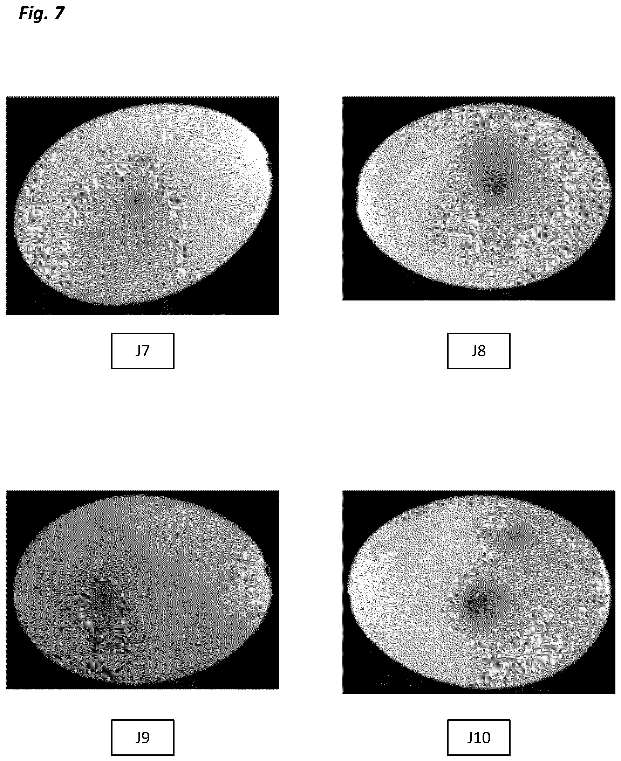

La figure 7 montre plusieurs photos prises par le capteur d'images C à différents stades d'incubation d'un œuf (à J7, J8, J9, J10), dans le cas de la première configuration décrite ci-dessus. La zone sombre représente le composant biologique contenant la mélanine absorbant le signal émis sur la plage de longueurs d'onde centrée sur 700nm. Dans le cas d'un canard mulard de sexe mâle, il s'agit de son œil (tache noire sur les images représentées). On constate ainsi que cet œil reste toujours parfaitement détectable, dès le 7^{ème} jour d'incubation et jusqu'au 10^{ème} jour.

Ainsi, en choisissant de filtrer les longueurs d'onde autour de 700nm, on obtient un contraste très important entre la mélanine et l'hémoglobine (rapport de 50 dans les coefficients d'absorption), ce qui fait parfaitement ressortir l'œil.

La solution de l'invention présente ainsi de nombreux avantages :
- Elle permet de caractériser un œuf de manière fiable, non-invasive, à un stade précoce de l'incubation ;
- Elle utilise des moyens simples et fiables ;
- Elle est adaptable aux installations déjà existantes ;

## Revendications

1. Procédé de caractérisation du genre de l'embryon d"un œuf (O) à partir de la présence d'un organe au sein de l'embryon de l'œuf, ledit procédé étant **caractérisé en ce qu'**il comporte des étapes de :
- Préparation de l'œuf, consistant à mettre l'œuf (O) en position couchée sur un support, l'axe longitudinal (X) de l'œuf étant parallèle audit support sur lequel il repose,
- Mise en attente de l'œuf (O) ainsi positionné pour que l'embryon de l'œuf migre vers le haut de l'œuf,
- Agitation mécanique de l'œuf par rotation de l'œuf autour de son axe longitudinal (X), en vue d'optimiser le positionnement dudit organe au plus près de la coquille,
- Pendant l'étape d'agitation mécanique, émission à travers l'œuf, par une source lumineuse (E), d'un flux lumineux, et acquisition de plusieurs images de l'œuf,
- Analyse des images de l'œuf (O) générées, la présence dudit organe étant révélée par contraste d'absorption entre les composantes constitutives de l'œuf (O) contenant du sang et ledit organe de l'embryon contenant de la mélanine.

2. Procédé selon la revendication 1, **caractérisé en ce qu'**il comporte une étape de filtrage du flux lumineux dans une bande spectrale étroite.

3. Procédé selon la revendication 2, **caractérisé en ce que** la bande spectrale étroite est comprise entre 650nm et 750nm.

4. Procédé selon la revendication 2, **caractérisé en ce que** la bande spectrale étroite est comprise entre 675nm et 725nm.

5. Procédé selon l'une des revendications 2 à 4, **caractérisé en ce que** l'étape de filtrage est mise en œuvre sur le flux lumineux émis par la source lumineuse (E), avant transmission dans l'œuf.

6. Procédé selon l'une des revendications 2 à 4, **caractérisé en ce que** l'étape de filtrage est mise en œuvre sur le flux lumineux émis par la source lumineuse (E), après transmission à travers l'œuf (O) et avant l'étape d'acquisition d'images de l'œuf.

7. Procédé selon l'une des revendications 1 à 6, **caractérisé en ce que** l'étape d'agitation mécanique consiste à mettre en œuvre une séquence de rotation de l'œuf autour de son axe longitudinal, ladite séquence consistant au moins à faire tourner l'œuf (O) sur lui-même autour de son axe longitudinal (X) dans un sens sur une première plage angulaire non nulle, puis dans le sens opposé sur une deuxième plage angulaire non nulle.

8. Procédé selon la revendication 7, **caractérisé en ce que** la séquence de rotation est mise en œuvre en jouant sur quatre paramètres :
- Le sens de rotation de l'œuf ;
- La vitesse de rotation de l'œuf ;
- L'accélération conférée lors de la rotation ;
- La plage angulaire de rotation de l'œuf ;

9. Système de caractérisation du genre de l'embryon d'un œuf (O) à partir de la présence d'un organe au sein de l'embryon de l'œuf, employé pour mettre en œuvre le procédé tel que défini dans l'une des revendications 1 à 8, **caractérisé en ce qu'**il comporte :
- Un poste de préparation (12) de l'œuf, configuré pour mettre l'œuf en position couchée sur un support, l'axe longitudinal (X) de l'œuf étant parallèle audit support sur lequel il repose,
- Un système (3) d'agitation mécanique de l'œuf par rotation de l'œuf autour de son axe longitudinal (X), en vue d'optimiser le positionnement dudit organe au plus près de la coquille,
- Un système d'imagerie comprenant une source lumineuse (E) apte à émettre un flux lumineux à travers l'œuf (O), et un dispositif d'acquisition de plusieurs images de l'œuf, le système d'imagerie étant actionné pour fournir des images de l'œuf pendant son agitation mécanique,
- Des moyens d'analyse des images de l'œuf (O) générées, la présence dudit organe étant révélée par contraste d'absorption entre les composantes constitutives de l'œuf (O) contenant du sang et ledit organe de l'embryon contenant de la mélanine.

10. Système selon la revendication 9, **caractérisé en ce qu'**il comporte des moyens de filtrage du flux lumineux émis par la source lumineuse (E) configurés pour filtrer ledit flux lumineux dans une bande spectrale étroite.

11. Système selon la revendication 10, **caractérisé en ce que** la bande spectrale étroite est comprise entre 650nm et 750nm.

12. Système selon la revendication 10 ou 11, **caractérisé en ce que** la bande spectrale étroite est comprise entre 675nm et 725nm.

13. Système selon l'une des revendications 10 à 12, **caractérisé en ce que** les moyens de filtrage sont agencés pour filtrer le flux lumineux émis par la source lumineuse (E), avant transmission dans l'œuf.

14. Système selon l'une des revendications 10 à 12, **caractérisé en ce que** les moyens de filtrage sont agencés pour filtrer le flux lumineux émis par la source lumineuse (E), après transmission à travers l'œuf (O) et avant acquisition d'images de l'œuf par le dispositif d'acquisition.

15. Système selon l'une des revendications 9 à 14, **caractérisé en ce que** le système d'agitation mécanique est contrôlé pour mettre en œuvre une séquence de rotation de l'œuf autour de son axe longitudinal, ladite séquence consistant au moins à faire tourner l'œuf (O) sur lui-même autour de son axe longitudinal (X) dans un sens sur une première plage angulaire non nulle, puis dans le sens opposé sur une deuxième plage angulaire non nulle.

16. Système selon la revendication 15, **caractérisé en ce que** la séquence de rotation est mise en œuvre en jouant sur quatre paramètres :
- Le sens de rotation de l'œuf ;
- La vitesse de rotation de l'œuf ;
- L'accélération conférée lors de la rotation ;
- La plage angulaire de rotation de l'œuf ;

## Patentansprüche

1. Verfahren zur Charakterisierung des Geschlechts des Embryos eines Eies (O) ausgehend von dem Vorhandensein eines Organs in dem Embryo des Eies, wobei das Verfahren **dadurch gekennzeichnet ist, dass** es die folgenden Schritte umfasst:
- Vorbereiten des Eies, das darin besteht, das Ei (O) in liegender Position auf einen Träger zu setzen, wobei die Längsachse (X) des Eies parallel zu dem Träger ist, auf dem es aufliegt;
- Ruhen lassen des so positionierten Eies (O), damit der Embryo des Eies zu der Oberseite des Eies wandert,
- Mechanisches Bewegen des Eies durch Drehen des Eies um seine Längsachse (X), um die Positionierung des Organs möglichst nah an der Schale zu optimieren;
- Während des Schritts des mechanischen Bewegens, Emittieren eines Lichtstroms durch eine Lichtquelle (E) durch das Ei hindurch und Erfassen von mehreren Bildern des Eies,
- Analysieren der erzeugten Bilder des Eies (O), wobei das Vorhandensein des Organs durch Absorptionskontrast zwischen den Blut enthaltenden Bestandteilen des Eies (O) und dem Melanin enthaltenden Organ des Embryos festgestellt wird.

2. Verfahren nach Anspruch 1, **dadurch gekennzeichnet, dass** es einen Schritt des Filterns des Lichtstroms in einem schmalen Spektralband umfasst.

3. Verfahren nach Anspruch 2, **dadurch gekennzeichnet, dass** das schmale Spektralband zwischen 650 nm und 750 nm liegt.

4. Verfahren nach Anspruch 2, **dadurch gekennzeichnet, dass** das schmale Spektralband zwischen 675 nm und 725 nm liegt.

5. Verfahren nach einem der Ansprüche 2 bis 4, **dadurch gekennzeichnet, dass** der Schritt des Filterns an dem von der Lichtquelle (E) emittierten Lichtstrom vor der Transmission in das Ei durchgeführt wird.

6. Verfahren nach einem der Ansprüche 2 bis 4, **dadurch gekennzeichnet, dass** der Schritt des Filterns an dem von der Lichtquelle (E) emittierten Lichtstrom nach der Transmission durch das Ei (O) hindurch und vor dem Schritt des Erfassens von Bildern des Eies durchgeführt wird.

7. Verfahren nach einem der Ansprüche 1 bis 6, **dadurch gekennzeichnet, dass** der Schritt des mechanischen Bewegens darin besteht, eine Drehsequenz des Eies um seine Längsachse durchzuführen, wobei die Sequenz mindestens darin besteht, das Ei (O) um sich selbst um seine Längsachse (X) in eine Richtung über einen ersten Winkelbereich ungleich Null und dann in die entgegengesetzte Richtung über einen zweiten Winkelbereich ungleich Null drehen zu lassen.

8. Verfahren nach Anspruch 7, **dadurch gekennzeichnet, dass** die Drehsequenz unter Einwirkung auf vier Parameter durchgeführt wird:
- Die Drehrichtung des Eies;
- Die Drehgeschwindigkeit des Eies;
- Die bei der Drehung verliehene Beschleunigung;
- Der Drehwinkelbereich des Eies.

9. System zur Charakterisierung des Geschlechts des Embryos eines Eies (O) ausgehend von dem Vorhandensein eines Organs in dem Embryo des Eies, das zur Durchführung des Verfahrens gemäß einem der Ansprüche 1 bis 8 eingesetzt wird, **dadurch gekennzeichnet, dass** es umfasst:
- Eine Vorbereitungsstation (12) für das Ei, die dazu ausgestaltet ist, das Ei liegend auf einen Träger zu setzen, wobei die Längsachse (X) des Eies parallel zu dem Träger ist, auf dem es aufliegt;
- Ein System (3) zum mechanischen Bewegen des Eies durch Drehen des Eies um seine Längsachse (X), um die Positionierung des Eies möglichst nah an der Schale zu optimieren;
- Ein Bildgebungssystem mit einer Lichtquelle (E), die geeignet ist, einen Lichtstrom durch das Ei (O) hindurch zu emittieren, und eine Vorrichtung zur Erfassung von mehreren Bildern des Eies, wobei das Bildgebungssystem so betätigt wird, dass es Bilder des Eies während des mechanischen Bewegens des Eies liefert;
- Mittel zum Analysieren der erzeugten Bilder des Eies (O), wobei das Vorhandensein des Organs durch Absorptionskontrast zwischen den Blut enthaltenden Bestandteilen des Eies (O) und dem Melanin enthaltenden Organ des Embryos festgestellt wird.

10. System nach Anspruch 9, **dadurch gekennzeichnet, dass** es Mittel zum Filtern des von der Lichtquelle (E) emittierten Lichtstroms beinhaltet, die dazu ausgestaltet sind, den Lichtstrom in einem schmalen Spektralband zu filtern.

11. System nach Anspruch 10, **dadurch gekennzeichnet, dass** das schmale Spektralband zwischen 650 nm und 750 nm liegt.

12. System nach Anspruch 10 oder 11, **dadurch gekennzeichnet, dass** das schmale Spektralband zwischen 675 nm und 725 nm liegt.

13. System nach einem der Ansprüche 10 bis 12, **dadurch gekennzeichnet, dass** die Mittel zum Filtern dazu eingerichtet sind, den von der Lichtquelle (E) emittierten Lichtstrom vor der Transmission in das Ei zu filtern.

14. System nach einem der Ansprüche 10 bis 12, **dadurch gekennzeichnet, dass** die Mittel zum Filtern dazu eingerichtet sind, den von der Lichtquelle (E) emittierten Lichtstrom nach der Transmission durch das Ei (O) hindurch und vor dem Erfassen von Bildern des Eies durch die Erfassungsvorrichtung zu filtern.

15. System nach einem der Ansprüche 9 bis 14, **dadurch gekennzeichnet, dass** das System zum mechanischen Bewegen so gesteuert wird, dass eine Drehsequenz des Eies um seine Längsachse durchgeführt wird, wobei die Sequenz mindestens darin besteht, das Ei (O) um sich selbst um seine Längsachse (X) in eine Richtung über einen ersten Winkelbereich ungleich Null und dann in die entgegengesetzte Richtung über einen zweiten Winkelbereich ungleich Null drehen zu lassen.

16. System nach Anspruch 15, **dadurch gekennzeichnet, dass** die Drehsequenz unter Einwirkung auf vier Parameter durchgeführt wird:
- Die Drehrichtung des Eies;
- Die Drehgeschwindigkeit des Eies;
- Die bei der Drehung verliehene Beschleunigung;
- Der Drehwinkelbereich des Eies.

## Claims

1. Method for characterizing the gender of the embryo of an egg (O) based on the presence of an organ within the embryo of the egg, said method being **characterized in that** it comprises steps of:
- preparing the egg, this consisting in placing the egg (O) on its side on a holder, the longitudinal axis (X) of the egg lying parallel to said holder on which the egg rests;
- waiting with the egg (O) thus positioned for the embryo of the egg to migrate to the top of the egg;
- carrying out mechanical agitation of the egg by rotating the egg about its longitudinal axis (X), so as to optimize the position of said organ by getting it as close as possible to the shell;
- during the step of carrying out mechanical agitation, emitting a light flux through the egg by means of a light source (E), and acquiring a plurality of images of the egg;
- analysing the generated images of the egg (O), the presence of said organ being revealed through a contrast in absorption between constituent components of the egg (O) that contain blood and said organ of the embryo, which contains melanin.

2. Method according to Claim 1, **characterized in that** it comprises a step of filtering the light flux in a narrow spectral band.

3. Method according to Claim 2, **characterized in that** the narrow spectral band is between 650 nm and 750 nm.

4. Method according to Claim 2, **characterized in that** the narrow spectral band is between 675 nm and 725 nm.

5. Method according to any of Claims 2 to 4, **characterized in that** the filtering step is implemented on the light flux emitted by the light source (E), before transmission into the egg.

6. Method according to any of Claims 2 to 4, **characterized in that** the filtering step is implemented on the light flux emitted by the light source (E), after transmission through the egg (O) and before the step of acquiring images of the egg.

7. Method according to any of Claims 1 to 6, **characterized in that** the step of carrying out mechanical agitation consists in implementing a sequence of rotation of the egg about its longitudinal axis, said sequence at least consisting in making the egg (O) rotate on itself about its longitudinal axis (X) in one direction over a first non-zero angular range, and then in the opposite direction over a second non-zero angular range.

8. Method according to Claim 7, **characterized in that** the rotation sequence is implemented with adjustment of four parameters:
- the direction of rotation of the egg;
- the speed of rotation of the egg;
- the acceleration imparted during the rotation;
- the angular range of rotation of the egg.

9. System for characterizing the gender of the embryo of an egg (O) based on the presence of an organ within the embryo of the egg, said system being employed to implement the method defined in any of Claims 1 to 8, and being **characterized in that** it comprises:
- a station (12) for preparing the egg, configured to place the egg on its side on a holder, the longitudinal axis (X) of the egg lying parallel to said holder on which the egg rests;
- a system (3) for carrying out mechanical agitation of the egg by rotating the egg about its longitudinal axis (X), so as to optimize the position of said organ by getting it as close as possible to the shell;
- an imaging system comprising a light source (E) suitable for emitting a light flux through the egg (O), and a device for acquiring a plurality of images of the egg, the imaging system being actuated so as to deliver images of the egg during its mechanical agitation;
- means for analysing the generated images of the egg (O), the presence of said organ being revealed through a contrast in absorption between constituent components of the egg (O) that contain blood and said organ of the embryo, which contains melanin.

10. System according to Claim 9, **characterized in that** it comprises means for filtering the light flux emitted by the light source (E), said means being configured to filter said light flux in a narrow spectral band.

11. System according to Claim 10, **characterized in that** the narrow spectral band is between 650 nm and 750 nm.

12. System according to Claim 10 or 11, **characterized in that** the narrow spectral band is between 675 nm and 725 nm.

13. System according to any of Claims 10 to 12, **characterized in that** the filtering means are arranged to filter the light flux emitted by the light source (E), before transmission into the egg.

14. System according to any of Claims 10 to 12, **characterized in that** the filtering means are arranged to filter the light flux emitted by the light source (E), after transmission through the egg (O) and before images of the egg are acquired by the acquiring device.

15. System according to any of Claims 9 to 14, **characterized in that** the system for carrying out mechanical agitation is controlled so as to implement a sequence of rotation of the egg about its longitudinal axis, said sequence at least consisting in making the egg (O) rotate on itself about its longitudinal axis (X) in one direction over a first non-zero angular range, and then in the opposite direction over a second non-zero angular range.

16. System according to Claim 15, **characterized in that** the rotation sequence is implemented with adjustment of four parameters:
- the direction of rotation of the egg;
- the speed of rotation of the egg;
- the acceleration imparted during the rotation;
- the angular range of rotation of the egg.
